# FASCICULE DE BREVET EUROPEEN

(11) **EP 3 801 214 B1**
(45) Date de publication et mention de la délivrance du brevet: **15.06.2022**
(21) Numéro de dépôt: 19742436.9
(22) Date de dépôt: 03.06.2019
(51) Int. Cl.: A61B 5/00

(54) **SONDE PHOTOACOUSTIQUE ENDOSCOPIQUE**
ENDOSKOPISCHE PHOTOAKUSTISCHE SONDE
ENDOSCOPIC PHOTOACOUSTIC PROBE

(30) Priorité: 01.06.2018 FR 1854804
(43) Date de publication de la demande: 14.04.2021
(73) Titulaire: Université Grenoble Alpes, 38400 Saint-Martin-d'Hères (FR)
(72) Inventeur: CUSTILLON, Guillaume, 38130 Echirolles (FR); DUBOIS, Nicolas, 38330 Montbonnot Saint Martin (FR)
(74) Mandataire: Regimbeau
(86) Numéro de dépôt international: PCT/FR2019/051294
(87) Numéro de publication internationale: WO 2019/229400

(56) Documents cités:
- WO-A1-01/22866
- WO-A1-2012/103903
- MIYA ISHIHARA ET AL: "Development of a diagnostic system for osteoarthritis using a photoacoustic measurement method", LASERS IN SURGERY AND MEDICINE., vol. 38, no. 3, 1 mars 2006 (2006-03-01), pages 249-255, XP055556405, US ISSN: 0196-8092, DOI: 10.1002/lsm.20285

## Description

### DOMAINE DE L'INVENTION

La présente invention concerne une sonde photoacoustique endoscopique.

### ETAT DE LA TECHNIQUE

L'imagerie médicale regroupe les moyens d'acquisition et de restitution d'images à partir de différents phénomènes physiques : résonance magnétique, réflexion d'ondes ultrasonores, radioactivité, absorption des rayons X... Ces technologies permettent de visualiser la physiologie ou le métabolisme du corps humain, d'établir un diagnostic précis et d'orienter les choix thérapeutiques. Cependant, elles représentent une panoplie d'imagerie imparfaite : certaines informations restent inaccessibles ou peu ergonomiques (notamment en n'étant pas en temps réel), et les irradiations sont reconnues comme un problème majeur pour les patients et le personnel soignant.

Seules certaines technologies sont disponibles au bloc opératoire selon le type de chirurgie. L'orthopédie requiert l'identification des structures osseuses et fait donc principalement appel à l'utilisation de rayons X.

L'utilisation de l'imagerie per-opératoire a pour but de donner des informations supplémentaires quant aux tissus lésés, d'augmenter la précision du geste opératoire, de réduire le temps d'intervention dans un contexte de chirurgie mini-invasive qui permet l'augmentation des bénéfices cliniques.

L'arthroscopie est une intervention chirurgicale mini-invasive au cours de laquelle une caméra miniature est introduite dans une articulation par une incision punctiforme et un instrument miniature est introduit par un deuxième orifice punctiforme. Le chirurgien peut donc réaliser un acte diagnostique en appréciant les structures anatomiques de la cavité articulaire et un acte thérapeutique adapté grâce à l'instrument approprié. Cette approche chirurgicale, réalisée en ambulatoire, a révolutionné la pratique chirurgicale en permettant d'opérer sans ouvrir et avec une diminution de la morbidité post-opératoire (diminution de la durée d'hospitalisation et de la période de réadaptation fonctionnelle).

Les articulations du genou et de l'épaule ont rapidement bénéficié de cette innovation chirurgicale. Tout ou partie des structures anatomiques constitutives de ces articulations peuvent être altérées lors d'un traumatisme ou d'une arthrose. Une arthroscopie du genou peut ainsi porter sur les ménisques (par exemple en vue d'une ablation), le cartilage (par exemple en vue d'une régularisation), la synoviale (par exemple pour une excision d'adhérence), la résection de petits fragments cartilagineux ou osseux (corps étrangers), ou certaines chirurgies plus importantes du genou (ligamentoplasties ou autres). De même, et de manière non exhaustive, une indication d'arthroscopie de l'épaule peut être posée pour la prise en charge de calcifications intratendineuses de la coiffe des rotateurs, d'un conflit sous-acromial traité en réalisant une bursectomie et acromioplastie sous arthroscopie, ou encore pour qualifier, en per-opératoire, une rupture de la coiffe des rotateurs.

Lors d'une arthroscopie, l'exploration visuelle de l'intérieur de la cavité articulaire se limite à la surface des structures constitutives de l'articulation, avec, comme conséquences, de potentielles limitations diagnostiques ou thérapeutiques. Par ailleurs, s'agissant d'une zone inaccessible, elle ne se prête pas à l'injection d'un agent de contraste ou d'un agent fluorescent pour faire apparaître la vascularisation des structures anatomiques.

Une sonde photoacoustique endoscopique a la capacité d'imager des structures anatomiques en profondeur mais aussi la vascularisation desdites structures et apporte ainsi des informations supplémentaires au chirurgien.

La publication par M. Ishihara et al: "Development of a diagnostic system for osteoarthritis using a photoacoustic measurement method", Lasers in surgery and medicine, vol. 38 no. 3, pages 249-255, divulgue une sonde échographique photoacoustique selon le préambule de la revendication 1.

Cependant, la configuration d'une articulation et l'espace disponible pour l'insertion d'une telle sonde rendent difficile l'utilisation de la photoacoustique en arthroscopie.

### EXPOSE DE L'INVENTION

Un but de l'invention est de concevoir une sonde photoacoustique endoscopique adaptée pour l'arthroscopie.

A cet effet, l'invention propose une sonde échographique photoacoustique, comprenant :
- un cathéter,
- un capteur échographique agencé à une extrémité distale du cathéter,
- au moins une fibre optique adaptée pour être reliée à une source laser, ladite fibre optique s'étendant dans le cathéter jusqu'à l'extrémité distale,
ladite sonde étant caractérisée en ce que le cathéter présente une portion distale inclinée et/ou une extrémité distale biseautée.

Dans le présent texte, les termes relatifs « proximal » et « distal » s'entendent respectivement d'une partie de la sonde située du côté de l'opérateur qui la manipule, et d'une partie de la sonde du côté du corps du patient dans lequel elle est destinée à être insérée.

Selon un mode de réalisation, le cathéter présente une portion distale inclinée d'un angle compris entre 10 et 30° par rapport à une portion proximale du cathéter.

Selon un mode de réalisation, l'extrémité distale est inclinée d'un angle compris entre 10 et 30° par rapport à un axe de révolution de la partie distale du cathéter.

De manière particulièrement avantageuse, l'extrémité distale du cathéter s'étend dans un plan incliné de 30° par rapport à la portion proximale du cathéter.

Selon un mode de réalisation préféré, la sonde comprend plusieurs fibres optiques agencées de part et d'autre du capteur échographique.

Selon une forme d'exécution, la sonde comprend en outre :
- une poignée de commande fixée à la portion proximale du cathéter,
- un connecteur adapté pour être connecté à une station échographique,
- un câble de connexion électrique s'étendant entre le connecteur et la poignée,
- une gaine comprenant une première portion entourant le cathéter, en un matériau adapté pour laisser passer les ultrasons et le faisceau laser, une seconde portion entourant la poignée et le câble jusqu'au connecteur, et un raccord agencé entre la première et la deuxième portion, ledit raccord étant fixé de manière amovible à la poignée.

Selon un mode de réalisation préféré, la première portion de la gaine est en un copolymère bloc polyamide-polyether.

De manière avantageuse, ladite première portion présente une épaisseur constante.

Selon un mode de réalisation, l'épaisseur de la première portion est comprise entre 0,4 et 0,6 mm.

De préférence, la seconde portion de la gaine présente un diamètre supérieur à celui de la première portion.

De manière avantageuse, le raccord comprend une ouverture centrale pour le passage du cathéter, la première portion étant fixée autour de ladite ouverture centrale du raccord et la seconde portion étant fixée à une portion périphérique du raccord.

Un autre objet de l'invention concerne un système d'imagerie comprenant une station échographique incluant une source laser, et une sonde photoacoustique endoscopique telle que décrite ci-dessus reliée à ladite station, dans lequel la station comprend un processeur configuré pour afficher une image de la vascularisation d'une zone d'intérêt intra-articulaire acquise par la sonde.

De manière particulièrement avantageuse, le processeur est configuré pour superposer une image échographique d'un ménisque et une image photoacoustique de la vascularisation dudit ménisque.

### DESCRIPTION DES FIGURES

D'autres caractéristiques et avantages de l'invention ressortiront de la description détaillée qui va suivre, en référence aux dessins annexés sur lesquels :
- la figure 1 est une vue en perspective du cathéter d'une sonde selon l'invention ;
- la figure 2 est une vue de côté dudit cathéter ;
- la figure 3 est une vue de face du cathéter ;
- la figure 4 est un schéma de principe de la sonde ;
- la figure 5 illustre la sonde de la figure 4 munie d'une gaine ;
- la figure 6 est une vue en perspective de la gaine seule ;
- la figure 7 est une vue schématique d'un montage de test de la sonde photoacoustique ;
- la figure 8 présente des images de la vascularisation d'un ménisque de mouton acquises avec la sonde photoacoustique ;
- la figure 9 illustre de manière schématique la vascularisation du ménisque humain selon les différentes zones ;
- la figure 10 est une vue schématique d'un premier montage de test qualitatif de la gaine ;
- les figures 11A-11D sont des images échographiques obtenues grâce au montage de la figure 10, respectivement en l'absence de gaine, avec une gaine en PEBD, avec une gaine en PEBAX^{™} 2533 et avec une gaine en PEBAX^{™} 3533;
- la figure 12 est une vue schématique d'un second montage de test qualitatif de la gaine ;
- la figure 13 est un graphique représentant l'atténuation globale (en dB) calculée par intégration des atténuations de la figure 12 sur l'ensemble des fréquences pour les différents matériaux testés ;
- les figures 14A et 14B sont des images échographiques d'un cartilage animal épais obtenues par une sonde respectivement dépourvue de gaine et protégée par une gaine en PEBAX^{™} 2533 ;
- les figures 15A et 15B sont des images échographiques d'un cartilage mince obtenues par une sonde respectivement dépourvue de gaine et protégée par une gaine en PEBAX^{™} 2533.

Les signes de référence identiques d'une figure à l'autre désignent des éléments identiques ou remplissant la même fonction.

### DESCRIPTION DETAILLEE DE MODES DE REALISATION DE L'INVENTION

La sonde photoacoustique endoscopique fait partie d'un système d'imagerie comprenant une station d'échographie à laquelle la sonde est reliée.

La station comprend une électronique de pilotage échographique, une source laser, un processeur adapté pour traiter les images acquises par la sonde, un écran de visualisation et un clavier permettant d'entrer des données et d'annoter les images.

Pendant une opération sous arthroscopie de l'épaule ou du genou, la sonde photoacoustique endoscopique est insérée dans l'articulation en utilisant la voie instrumentale. La sonde est toujours utilisée avec l'arthroscope qui utilise l'autre voie. Le chirurgien amène la partie distale de la sonde dans la zone d'intérêt en s'aidant de l'arthroscope et inspecte les structures anatomiques dans cette zone en orientant la sonde vers celles-ci.

La sonde photoacoustique comprend à la fois un capteur échographique qui permet de réaliser des images échographiques dans l'articulation et une ou plusieurs fibres optiques reliées à la source laser pour acquérir des images photoacoustiques, qui peuvent être combinées ou non avec les images échographiques. En effet, l'excitation par un faisceau laser d'une structure anatomique contenant de l'hémoglobine génère une onde ultrasonore qui est enregistrée par le capteur échographique pour fournir une image photoacoustique. Le capteur échographique peut en outre émettre une onde ultrasonore et recueillir l'onde ultrasonore réfléchie par les structures anatomiques pour fournir une image échographique. L'image photoacoustique peut être superposée à l'image échographique.

La fréquence des ultrasons est avantageusement comprise dans la gamme allant de 5 à 60 Mhz.

La longueur d'onde du faisceau laser est avantageusement comprise entre 500 et 1200 nm.

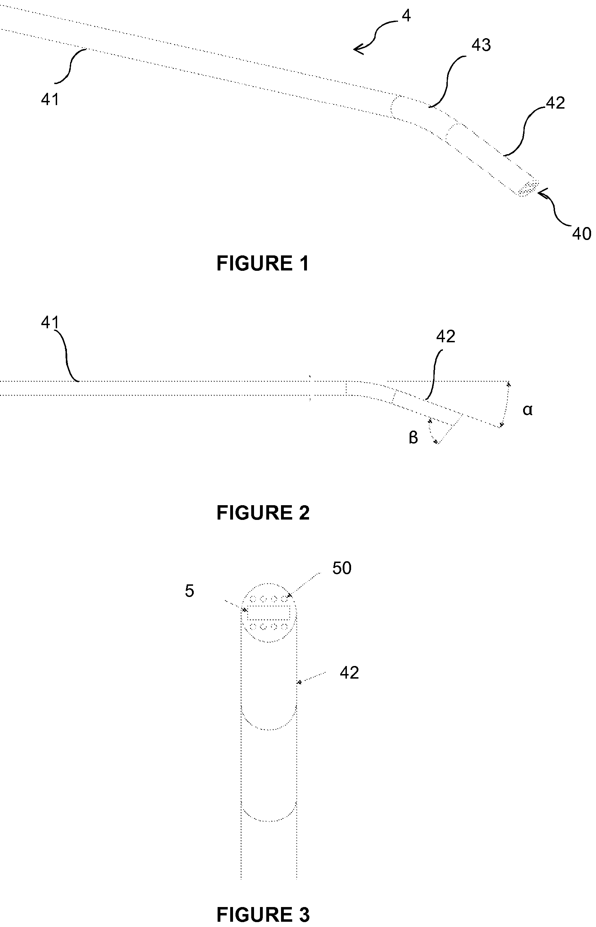

Les figures 1 à 3 sont des vues d'un cathéter d'une sonde selon l'invention.

Le cathéter 4 inclut un capteur échographique 5 et au moins une fibre optique 50 qui s'étend le long du cathéter jusqu'à l'extrémité distale 40. L'extrémité distale du cathéter comprend une lentille en regard de ladite fibre optique.

De préférence, le cathéter inclut plusieurs fibres optiques, par exemple de 6 à 12 fibres optiques, afin d'augmenter le signal lumineux d'excitation.

Le capteur échographique comprend avantageusement un transducteur multi-éléments agencés sous la forme d'un réseau linéaire, par exemple au nombre de 15 à 25 éléments.

De manière avantageuse, le capteur échographique 5 est agencé à l'extrémité distale 40 du cathéter. Les fibres optiques 50 sont de préférence agencées de part et d'autre du capteur échographique.

Le cathéter présente une longueur généralement comprise entre 10 et 20 cm et un diamètre de quelques millimètres, par exemple 4 mm. Le cathéter peut être en inox ou en titane.

Le cathéter présente une portion distale 42 inclinée et/ou une extrémité distale 40 biseautée. Par « portion distale inclinée » on entend que la portion distale 42 du cathéter est inclinée par rapport à la portion proximale 41 du cathéter (angle α sur la figure 2), les portions distales et proximales, qui sont droites, étant reliées par une portion courbe 43. Par « extrémité distale biseautée » on entend que le plan de l'extrémité distale 40 est incliné par rapport à l'axe de révolution de la portion distale 42 du cathéter (angle β sur la figure 2). Les angles d'inclinaison α, β sont typiquement de l'ordre d'une dizaine à une trentaine de degrés et peuvent être combinés.

Ainsi, une configuration du cathéter avec une inclinaison de l'extrémité distale du cathéter de 30° par rapport à la portion proximale est particulièrement avantageuse en arthroscopie, car elle permet de mieux s'adapter à la géométrie de l'articulation à inspecter. Par exemple, le cathéter peut présenter une portion distale inclinée de 10° par rapport à la portion proximale du cathéter, et une extrémité biseauté d'un angle de 20° par rapport à l'axe de révolution du cathéter.

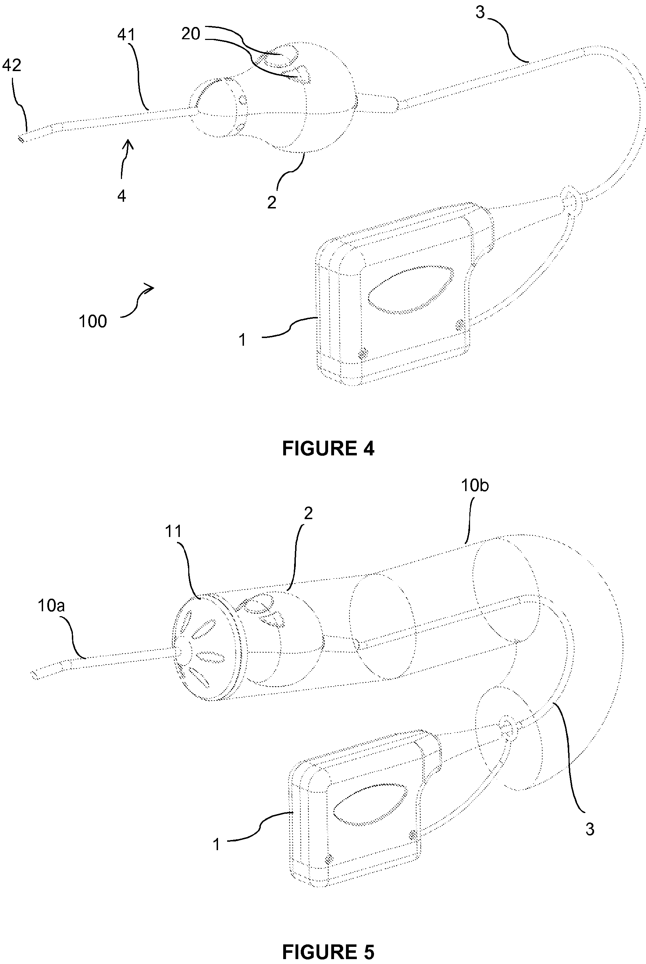

La figure 4 est un schéma de principe de la sonde.

La sonde 100 comprend un connecteur 1 par lequel elle peut être connectée à la station d'échographie (qui n'est pas représentée).

Le connecteur 1 est relié à une poignée 2 de commande par l'intermédiaire d'un câble 3 de connexion électrique. La poignée de commande est l'organe que l'utilisateur tient dans sa main lors de l'intervention. La poignée comprend un ou plusieurs boutons 20 que l'utilisateur peut presser par exemple pour naviguer ou pour valider l'acquisition d'une image ou d'une vidéo.

La portion proximale 41 du cathéter 4 est fixée à la poignée 2.

Selon un mode de réalisation avantageux, la sonde est protégée par une gaine stérile qui la recouvre entièrement, de l'extrémité distale du cathéter jusqu'au connecteur qui la relie à la station d'échographie. La gaine est d'un seul tenant et ne présente une ouverture qu'au niveau du connecteur.

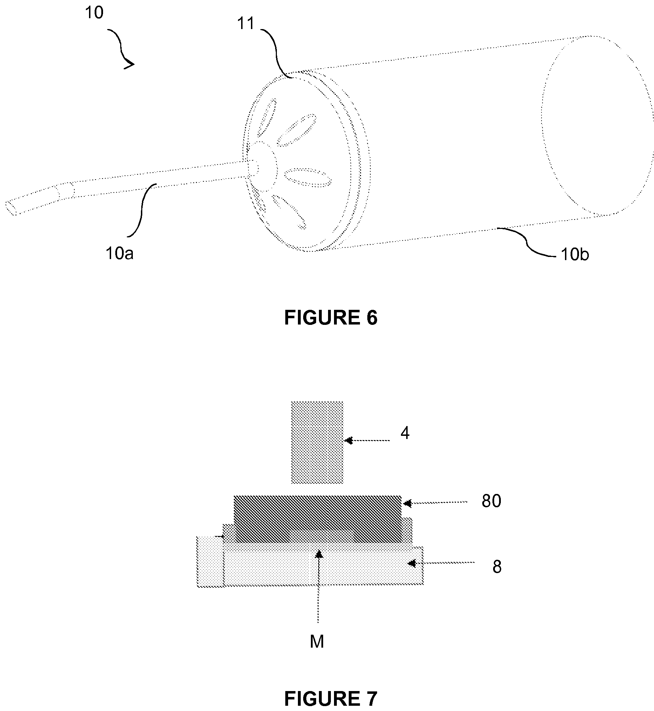

La figure 5 est un schéma de la sonde photoacoustique endoscopique équipée d'une telle gaine, et la figure 6 représente la gaine seule

La gaine 10 présente une première partie 10a adaptée pour entourer le cathéter, et une seconde partie 10b, plus large, entourant la poignée et le câble de connexion. Les dimensions de la gaine sont choisies pour s'adapter à la forme et aux dimensions de la sonde. D'une manière générale, la première et la seconde partie présentent chacune une forme cylindrique de section circulaire.

La première partie 10a épouse la forme du cathéter 4 au plus près afin de ne pas augmenter excessivement l'encombrement du cathéter, un léger jeu étant ménagé pour faciliter l'enfilage de la gaine sur le cathéter. Par exemple, pour une gaine de 0,5 mm d'épaisseur et un cathéter de 4 mm de diamètre, le diamètre extérieur de la première portion est de l'ordre de 5,4 mm. La première portion 10a de la gaine est fermée à son extrémité distale, y compris en regard de la lentille. La première portion 10a est suffisamment flexible compte tenu de son matériau et de son épaisseur pour s'adapter à la forme et à la courbure du cathéter le cas échéant.

La première et la seconde partie de la gaine sont reliées par un raccord 11 grâce auquel la gaine peut être fixée de manière amovible à la poignée de la sonde.

Le raccord est typiquement en un matériau thermoplastique.

La première portion 10a de la gaine est en un matériau adapté pour laisser passer le faisceau laser et le signal échographique avec une atténuation suffisamment faible pour que l'imagerie soit réalisable avec une résolution suffisante. Le choix d'un matériau adapté pour cette fonction sera décrit en détail plus bas. De préférence, la première portion de la gaine est en un copolymère bloc polyamide-polyether commercialisé notamment sous la dénomination PEBAX^{™}. De préférence, le PEBAX^{™} 2533 est préféré, le PEBAX^{™} 3533 étant satisfaisant mais moins préféré. L'épaisseur de la première portion de la gaine est choisie suffisamment fine pour ne pas engendrer une atténuation trop importante des ultrasons. Par exemple, l'épaisseur de cette première portion est comprise entre 0,4 et 0,6 mm, de manière préférée 0,5 mm. Cette épaisseur est avantageusement constante sur toute la longueur de la première portion.

La seconde portion 10b de la gaine peut être réalisée dans le même matériau que la première portion ou dans un autre matériau, cette seconde portion n'étant pas destinée à laisser passer les ultrasons. La seconde portion ne comporte pas nécessairement de moyen de fixation au connecteur. Elle est en effet suffisamment longue pour recouvrir la sonde jusqu'à la zone non stérile du site opératoire, même si elle est simplement enfilée autour du câble de connexion.

Le raccord 11 présente une ouverture centrale pour le passage du cathéter 4, autour de laquelle il s'étend radialement, la première portion 10a de la gaine étant fixée à une portion centrale du raccord, autour de ladite ouverture 110, et s'étend du côté distal du raccord ; la seconde portion 10b de la gaine est fixée à une portion périphérique du raccord et s'étend du côté proximal du raccord. La première et la deuxième portion 10a, 10b sont fixées de manière étanche au raccord 11 par tout moyen approprié, par exemple par collage sur le raccord ou par surmoulage du raccord.

Le raccord 11 et la poignée 2 présentent avantageusement des moyens mutuels de solidarisation réversible. Selon un mode de réalisation, la solidarisation est effectuée par un mécanisme quart de tour. De manière alternative, la solidarisation peut être effectuée par encliquetage.

Le(s) matériau(x) formant les deux portions de la gaine et le raccord sont stérilisables par des radiations ionisantes sans se dégrader. De préférence, la stérilisation est effectuée par une exposition à de l'oxyde d'éthylène suivie par une exposition à des rayons y.

La gaine 10 étant amovible vis-à-vis du reste de la sonde et recouvrant entièrement la partie de la sonde située dans le champ opératoire, elle peut être stérilisée isolément et permet de procurer un dispositif photoacoustique endoscopique stérile sans risquer d'endommager les composants fonctionnels de la sonde. Typiquement, la gaine est stérilisée avant d'être emballée dans un emballage adapté pour préserver la stérilité et n'est extraite de cet emballage que pour être mise en place sur la sonde.

On va maintenant décrire les modalités de choix du matériau de la première portion de la gaine.

Les inventeurs ont testé différents matériaux transparents aux ondes ultrasonores et les ont évalués qualitativement et quantitativement pour vérifier leur capacité à laisser passer le signal ultrasonore avec une atténuation suffisamment faible. Dans la mesure où le problème du matériau de la gaine se pose principalement pour le signal ultrasonore, la gaine a été testée pour une sonde échographique sans mettre en oeuvre d'excitation laser.

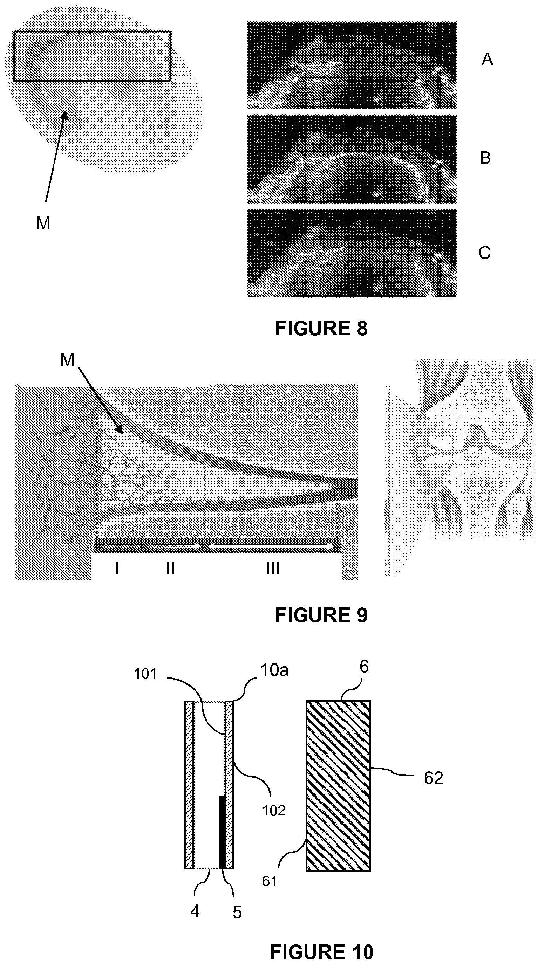

La figure 10 est un schéma de principe d'un premier montage pour un test qualitatif.

Le capteur échographique 5 de la sonde est agencé en regard d'une paroi 6 en PVC, l'ensemble étant immergé dans de l'eau.

Une première image échographique est acquise sans la gaine, puis une gaine 10a constituée de chacun des matériaux à tester est enfilée sur le cathéter 4, et une image échographique respective est acquise.

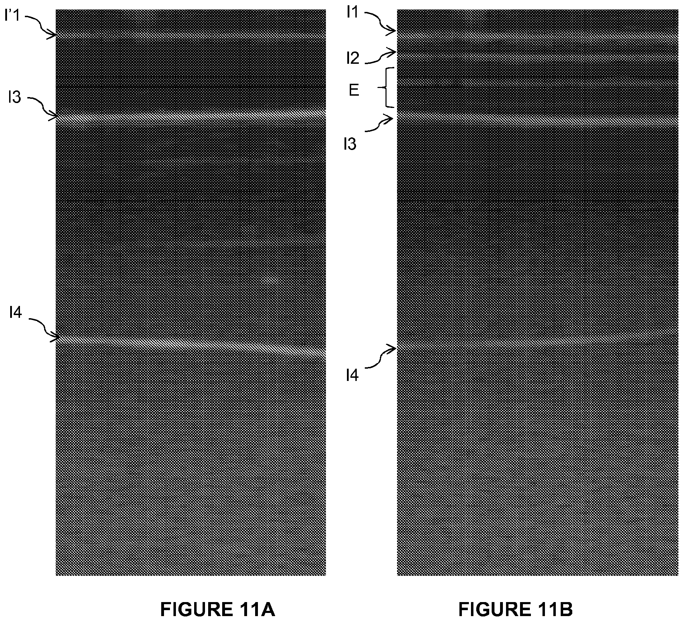

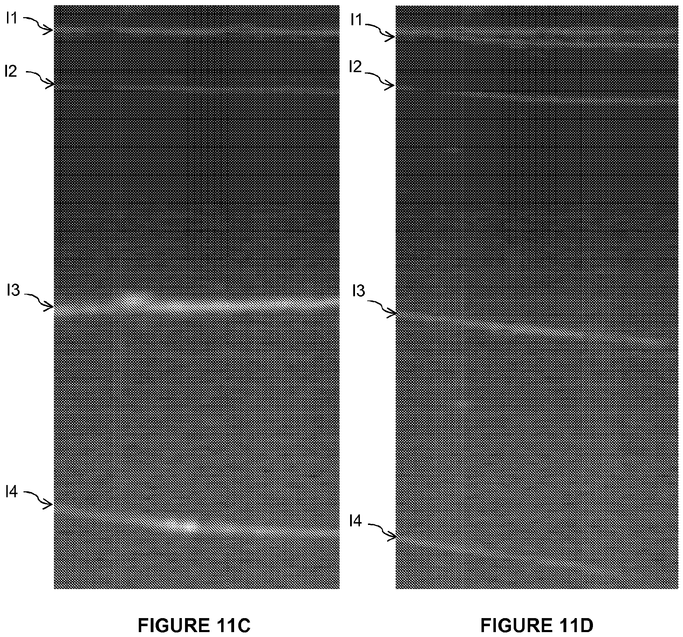

Les résultats présentés sur les figures 11A-11D sont respectivement obtenus sans gaine, avec une gaine en PEBD, avec une gaine en PEBAX^{™} 2533 (matériau préféré) et en PEBAX^{™} 3533. Les lignes blanches désignées par l1, l2, l3 et l4 correspondent respectivement à l'interface entre la lentille et la gaine, l'interface entre la gaine et l'eau, l'interface entre l'eau et le PVC (surface de la paroi en regard de la sonde) et l'interface entre le PVC (surface de la paroi opposée à la sonde) et l'eau. Dans le cas de la figure 11A, en l'absence de gaine, l'interface entre la lentille et l'eau est désignée par l'1.

Un premier critère de qualification d'un matériau envisagé pour la gaine est la visibilité de ces interfaces. On cherche à obtenir une visibilité aussi proche que possible que la visibilité obtenue sans gaine. La perte de visibilité des interfaces 61, 62 de la paroi PVC est due à l'absorption des ultrasons dans la gaine et à l'énergie réfléchie par les surfaces intérieure 101 et extérieure 102 de la gaine.

Par ailleurs, les éventuelles lignes désignées par E correspondent à des interfaces parasites dues à un phénomène de cavité dans l'épaisseur de la gaine. Ce phénomène est causé par une différence d'impédance acoustique entre la gaine et l'eau. De ce fait, les ultrasons font des allers-retours multiples entre la surface externe de la gaine et la sonde, chaque aller-retour produisant un écho qui se traduit par une ligne horizontale sur l'image. De telles interfaces parasites nuisent à la qualité de l'image. Ces échos parasites sont d'autant plus problématiques que dans l'application intra-articulaire visée, la sonde doit être très proche des tissus à échographier, voire au contact de ceux-ci, ce qui implique que les éventuels échos parasites se superposeront aux structures anatomiques à observer.

La présence et le nombre d'échos parasites constitue donc un deuxième critère de qualification du matériau. De tels échos doivent être évités ou tout au moins minimisés.

On observe sur ces images que la gaine en PEBD produit des échos parasites, bien que les interfaces attendues présentent une bonne visibilité. La gaine en PEBAX^{™} 2533, qui est le matériau le plus performant, ne génère pas d'échos parasites tout en procurant des interfaces bien visibles. Le PEBAX^{™} 3533 apparaît également comme un matériau relativement satisfaisant.

Le tableau ci-dessous récapitule les matériaux testés et leur classification en termes de nombre d'échos parasites et de visibilité des interfaces.

| Matériau de la gaine | Nombre d'échos parasites | Visibilité des interfaces |
|---|---|---|
| Sans gaine | 0 | +++ |
| PA12 | 4 | + |
| PEBD | 2 | ++ |
| PVC | 2 | + |
| Silicone | 0 | - |
| TPU95 | 2 | - |
| TPU98 | 3 | - |
| PEBAX^{™} 2533 | 0 | ++ |
| PEBAX^{™} 3533 | 0 | + |
| PEBAX^{™} 4533 | 0 | - |

Une autre étude a été menée pour évaluer quantitativement l'atténuation causée par les différents matériaux envisagés pour la gaine.

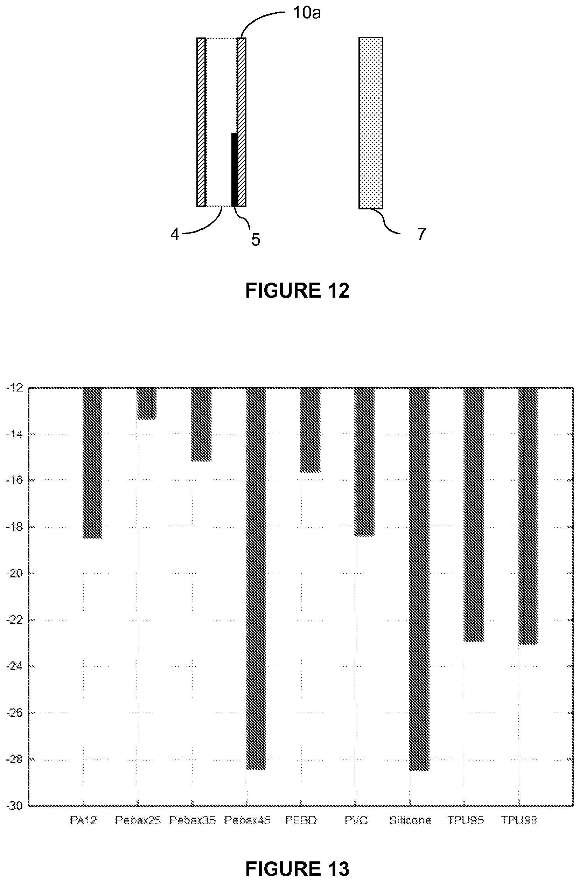

La figure 12 est un schéma de principe d'un second montage pour un test qualitatif.

Le capteur échographique 5 de la sonde est agencé en regard d'une paroi 7 en métal, qui est un bon réflecteur des ultrasons, l'ensemble étant fixé par un banc pour maintenir une distance constante entre la sonde et la paroi métallique.

Une première image échographique est acquise sans la gaine, puis une gaine 10a constituée de chacun des matériaux à tester est enfilée sur le cathéter 4, et une image échographique respective est acquise. Un calcul d'atténuation est alors réalisé.

La figure 13 est un graphique représentant l'atténuation globale (c'est-à-dire intégrée sur l'ensemble de la plage de fréquence d'intérêt, qui est comprise entre 10 et 15 MHz) (en dB) pour les différents matériaux testés.

On constate que le silicone et le PEBAX^{™} 4533 présentent une forte absorption, et ne sont donc pas préférés, bien qu'ils ne génèrent pas d'échos parasites. Le PEBAX^{™} 2533 est le meilleur candidat, avec une absorption d'environ 13 dB, suivi du PEBAX^{™} 3533 (15 dB) et du PEBD (15,5 dB). Cependant, le PEBD ne sera pas retenu en raison des échos parasites qu'il génère.

En conclusion, le PEBAX^{™} 2533 est le matériau testé qui présente le moins d'atténuation. De plus, il est important de noter que les gaines en PEBAX^{™} testées étaient plus épaisses (environ 2 mm d'épaisseur) que les autres (environ 0,8 mm). Ainsi, dans des conditions d'épaisseur identiques, une gaine en PEBAX^{™} 2533 présenterait encore moins d'atténuation que les autres.

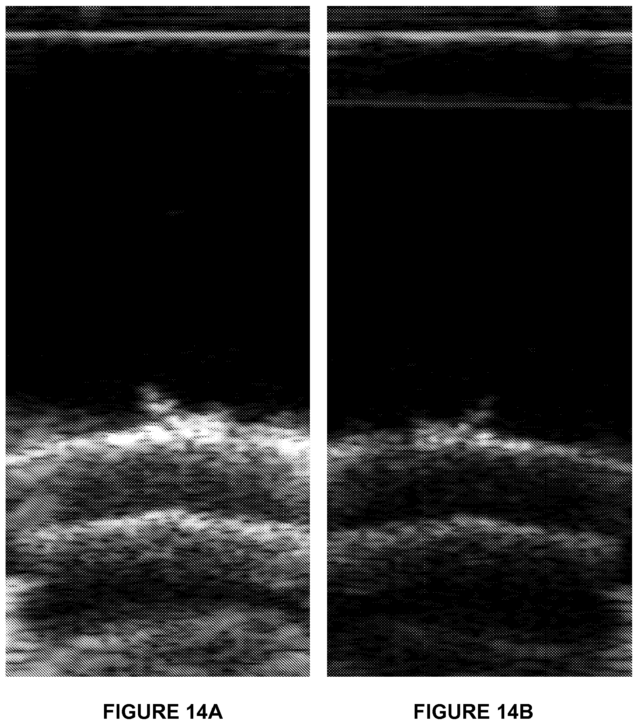

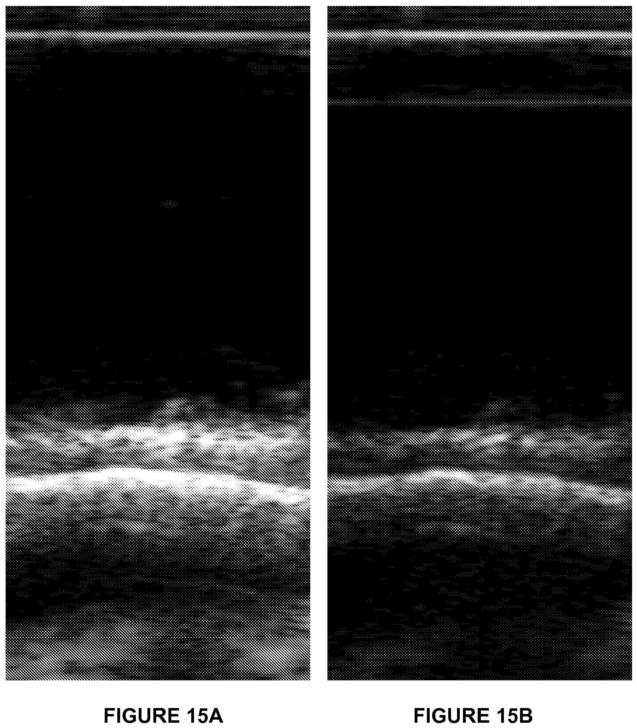

Pour valider que le PEBAX^{™} 2533 correspond bien aux attentes, des images de cartilage ont été réalisées en maintenant fixe la sonde sur des pièces anatomiques animales. Toutes les images ont été enregistrées avec les mêmes réglages de paramètres. Des images de cartilage épais (c'est-à-dire de 2,6 mm d'épaisseur environ) (cf. figures 14A-14B) et mince (c'est-à-dire de 1,6 mm d'épaisseur environ) (cf. figures 15A-15B) ont été enregistrées (visibles dans la partie inférieure des images). Les interfaces délimitant le cartilage sont toujours visibles avec la gaine en PEBAX^{™} 2533.

Lorsque la sonde est à nu dans l'eau, les interfaces sont très brillantes et saturent l'image : il faudrait diminuer le gain pour les observer correctement. Avec la gaine en PEBAX^{™} 2533, la saturation disparaît, comme si le gain avait été diminué à un niveau acceptable.

D'un point de vue acoustique, le PEBAX^{™} 2533 apparaît donc comme le meilleur matériau : son impédance acoustique est proche de celle de l'eau (pas d'échos parasites dus au phénomène de cavité) et il absorbe peu les ultrasons. Par conséquent, les images obtenues au travers de cette gaine présentent une qualité suffisante pour une visualisation en profondeur des structures anatomiques d'intérêt, tout en assurant la stérilité de la sonde.

De plus, le PEBAX^{™} 2533 est un matériau translucide, au travers duquel on peut voir l'intérieur de la sonde, notamment la position du capteur échographique sur les images arthroscopiques, ce qui permet de faciliter l'utilisation de la sonde par un chirurgien. Ce matériau est donc transparent au faisceau laser et permet donc l'émission du faisceau laser et l'émission/réception d'ondes échographiques.

La figure 7 est un schéma d'un montage de test de la sonde photoacoustique. Un ménisque frais de mouton (désigné par le repère M), présentant encore des traces d'hémoglobine, a été placé sur un porte-échantillon 8 et fixé dans de l'agarose 80. La sonde photoacoustique (seul le cathéter 4 est représenté) a été mise en place au-dessus de l'échantillon et des images photoacoustiques du ménisque ont été acquises. La longueur d'onde de la source laser était de 532 nm, et la fréquence ultrasonore de 15 MHz.

La figure 8 présente des images de la vascularisation d'un ménisque de mouton acquises avec la sonde photoacoustique. L'image échographique est une image moyenne sur l'ensemble de l'épaisseur du ménisque, correspondant à la zone encadrée sur la photographie du ménisque.

Sur l'image A, c'est le maximum des valeurs du signal photoacoustique sur l'épaisseur du ménisque qui a été reportée sur l'image échographique.

Sur l'image B, c'est la moyenne des valeurs du signal photoacoustique sur l'épaisseur du ménisque qui a été reportée sur l'image échographique.

Sur l'image C, ce sont les valeurs du signal photoacoustique sur l'épaisseur du ménisque supérieures à un seuil qui ont été reportées sur l'image échographique, afin d'éliminer le signal photoacoustique à l'extérieur du volume (correspondant à du bruit).

Ces images montrent la présence de signal photoacoustique sur la zone extérieure du ménisque, provenant probablement de l'hémoglobine contenue dans les vaisseaux du ménisque. C'est dans cette zone extérieure que l'on s'attend à avoir le plus de vascularisation, comme le montre la figure 9.

Les ménisques sont des fibrocartilages semi-lunaires interposés entre les condyles fémoraux et les plateaux tibiaux. Ils possèdent un ancrage par leurs cornes antérieures et postérieures au niveau des surfaces pré et rétrospinales du tibia, ainsi qu'un ancrage capsuloligamentaire circonférentiel par leur bord périphérique. Ce sont des structures mobiles et déformables subissant d'importantes forces en compression, cisaillement et torsion pouvant aboutir à des lésions diverses. Leur fonction est désormais connue : répartition des contraintes, stabilisation du genou sur la translation antérieure, nutrition et lubrification de l'articulation et proprioception.

Les lésions des ménisques sont fréquentes et touchent l'ensemble de la population. Elles peuvent être traumatiques et/ou dégénératives. Elles provoquent des douleurs, des blocages et des épanchements articulaires. Leur traitement est simple et consiste en une résection ou une réparation du ménisque.

Le résultat à moyen et long terme d'une méniscectomie, qui consiste en une ablation du ménisque (avec un développement possible d'arthrose secondaire), et les résultats des reconstructions du ligament croisé antérieur incitent les praticiens à préserver le ménisque. En effet, lorsqu'il est retiré, son absence contribue à une accélération de la dégradation de l'articulation, et à l'apparition d'arthrose précoce, en particulier chez les pratiquants de sports (ski, course à pieds, football...), y compris les jeunes. La préservation du ménisque permet de prévenir la dégradation du genou, et retarder le plus longtemps possible l'implantation d'une prothèse totale de genou. À l'heure actuelle, la seule technologie proposée en cas de lésion du ménisque est la suture, or la littérature montre un taux d'échec jusqu'à 43% (Pujol et al, "Amount of meniscal resection after failed meniscal repair", Am. J. Sports Med, 2011). Un des critères majeurs pour l'établissement d'un pronostic de succès est la visualisation de capillaires sanguins (constituant une micro-vascularisation) au niveau de la zone lésée. Cette micro-vascularisation n'est à l'heure actuelle pas détectable en conditions opératoires, ni en imagerie pré-opératoire. Les lésions étant dans plus de 50% des cas situées dans la zone d'incertitude, la décision du chirurgien penche dans la majorité des cas vers une ablation plutôt que vers une réparation à l'issue incertaine. Par ailleurs, du fait du coût élevé des crochets de suture, les quantités mises en dépôt dans les blocs opératoires ainsi que leur utilisation font l'objet d'une utilisation contrôlée.

La figure 9 est une vue en coupe schématisant les différentes zones de micro-vascularisation du ménisque. La vascularisation méniscale s'effectue à partir de la périphérie du ménisque (mur méniscal) alors que le bord libre du ménisque n'est pas vascularisé. On peut distinguer trois zones : la zone I (également appelée zone rouge) est la plus vascularisée ; la zone II (également appelée zone rouge-blanche) est moyennement vascularisée ; enfin, la zone III (également appelée zone blanche) n'est pas vascularisée. En pratique, une suture effectuée dans la zone I a de fortes chances de cicatriser parfaitement ; en revanche, une suture effectuée dans la zone II présente des chances de cicatrisation aléatoires. Enfin, une suture effectuée dans la zone III est vouée à l'échec du fait de l'absence de vascularisation.

La réparation méniscale est possible si la lésion méniscale siège près de l'insertion périphérique du ménisque et qu'elle se situe dans le plan vertical longitudinal. Cette suture se fait selon les mêmes modalités générales que la régularisation méniscale. Techniquement la mise en place d'une ancre résorbable permet d'amarrer les fils de suture. La réparation méniscale est surtout indiquée chez les sujets jeunes et les sujets ayant présenté une lésion méniscale suite à une rupture du ligament croisé antérieur. La suture méniscale sur genou stabilisé permettra alors une évolution et une cicatrisation favorable dans les meilleures conditions non seulement du ligament croisé mais aussi de l'appareil méniscal.

La sonde photoacoustique endoscopique procure au chirurgien un outil per-opératoire d'aide à la décision permettant de déterminer, avec un haut niveau de certitude, si, en fonction de la micro-vascularisation, la suture peut être réalisée avec succès.

La photoacoustique allie en effet deux avantages : d'une part la possibilité d'exciter spécifiquement l'hémoglobine (caractéristique de la cible que sont les capillaires sanguins), et d'autre part la possibilité de disposer d'une sensibilité suffisante pour détecter ces excitations dans la profondeur de la structure anatomique considérée (par exemple ménisque en fibrocartilage). L'utilisation de la sonde est un acte de diagnostic complémentaire aux pratiques actuelles qui s'inscrit dans le déroulement de l'acte chirurgical car il donne une information supplémentaire pour orienter la décision entre réparation ou ablation du ménisque.

Pour les mêmes raisons, la sonde photoacoustique endoscopique présente également un intérêt dans le traitement des lésions de la coiffe des rotateurs dans l'épaule. En effet, la vascularisation de la coiffe des rotateurs est l'un des éléments clés de la cicatrisation tendineuse après réparation des lésions.

## Revendications

1. Sonde échographique photoacoustique (100), comprenant :
- un cathéter (4),
- un capteur échographique (5) agencé à une extrémité distale (40) du cathéter,
- au moins une fibre optique (50) adaptée pour être reliée à une source laser, ladite fibre optique s'étendant dans le cathéter jusqu'à l'extrémité distale (40),
ladite sonde étant **caractérisée en ce que** le cathéter présente une portion distale (42) inclinée et/ou une extrémité distale (40) biseautée.

2. Sonde selon la revendication 1, dans laquelle le cathéter (4) présente une portion distale (42) inclinée d'un angle compris entre 10 et 30° par rapport à une portion proximale (41) du cathéter.

3. Sonde selon l'une des revendications 1 ou 2, dans laquelle l'extrémité distale (40) est inclinée d'un angle compris entre 10 et 30° par rapport à un axe de révolution de la partie distale (42) du cathéter.

4. Sonde selon les revendications 2 et 3 prises en combinaison, dans laquelle l'extrémité distale (40) du cathéter s'étend dans un plan incliné de 30° par rapport à la portion proximale (41) du cathéter.

5. Sonde selon l'une des revendications 1 à 4, comprenant plusieurs fibres optiques (50) agencées de part et d'autre du capteur échographique (5).

6. Sonde selon l'une des revendications 1 à 5, comprenant en outre :
- une poignée de commande (2) fixée à la portion proximale (41) du cathéter,
- un connecteur (1) adapté pour être connecté à une station échographique,
- un câble (3) de connexion électrique s'étendant entre le connecteur (1) et la poignée (2),
- une gaine (10) comprenant une première portion (10a) entourant le cathéter (4), en un matériau adapté pour laisser passer les ultrasons et le faisceau laser, une seconde portion (10b) entourant la poignée (2) et le câble (3) jusqu'au connecteur (1), et un raccord (11) agencé entre la première et la deuxième portion, ledit raccord étant fixé de manière amovible à la poignée (2).

7. Sonde selon la revendication 6, dans laquelle la première portion (10a) est en un copolymère bloc polyamide-polyether.

8. Sonde selon l'une des revendications 6 ou 7, dans laquelle la première portion (10a) présente une épaisseur constante.

9. Sonde selon l'une des revendications 6 à 8, dans laquelle l'épaisseur de la première portion (10a) est comprise entre 0,4 et 0,6 mm.

10. Sonde selon l'une des revendications 6 à 9, dans laquelle la seconde portion (10b) présente un diamètre supérieur à celui de la première portion (10a).

11. Sonde selon l'une des revendications 6 à 10, dans laquelle le raccord comprend une ouverture centrale pour le passage du cathéter, la première portion étant fixée autour de ladite ouverture centrale du raccord et la seconde portion étant fixée à une portion périphérique du raccord.

12. Système d'imagerie comprenant une station échographique incluant une source laser, et une sonde photoacoustique endoscopique selon l'une des revendications 1 à 11 reliée à ladite station, dans lequel la station comprend un processeur configuré pour afficher une image de la vascularisation d'une zone d'intérêt intra-articulaire acquise par la sonde.

13. Système selon la revendication 12, dans lequel le processeur est configuré pour superposer une image échographique d'un ménisque et une image photoacoustique de la vascularisation dudit ménisque.

## Patentansprüche

1. Photoakustische echographische Sonde (100), umfassend:
- einen Katheter (4),
- einen echographischen Sensor (5), der an einem distalen Ende (40) des Katheters angeordnet ist,
- mindestens eine optische Faser (50), die dazu geeignet ist, mit einer Laserquelle verbunden zu werden, wobei sich die optische Faser durch den Katheter bis zum distalen Ende (40) erstreckt, wobei die Sonde **dadurch gekennzeichnet ist, dass** der Katheter einen geneigten distalen Abschnitt (42) und/oder ein abgeschrägtes distales Ende (40) aufweist.

2. Sonde nach Anspruch 1, wobei der Katheter (4) einen distalen Abschnitt (42) aufweist, der in einem Winkel zwischen 10 und 30° zu einem proximalen Abschnitt (41) des Katheters geneigt ist.

3. Sonde nach einem der Ansprüche 1 oder 2, wobei das distale Ende (40) um einen Winkel zwischen 10 und 30° in Bezug auf eine Drehachse des distalen Teils (42) des Katheters geneigt ist.

4. Sonde nach den Ansprüchen 2 und 3 in Kombination, wobei das distale Ende (40) des Katheters sich in einer Ebene erstreckt, die um 30° gegen den proximalen Abschnitt (41) des Katheters geneigt ist.

5. Sonde nach einem der Ansprüche 1 bis 4, die mehrere optische Fasern (50) umfasst, die auf beiden Seiten des echographischen Sensors (5) angeordnet sind.

6. Sonde nach einem der Ansprüche 1 bis 5, ferner umfassend:
- einen Steuergriff (2), der am proximalen Abschnitt (41) des Katheters befestigt ist,
- ein Verbindungsstück (1), das für den Anschluss an eine Ultraschallstation geeignet ist,
- ein elektrisches Verbindungskabel (3), das sich zwischen dem Verbindungsstück (1) und dem Steuergriff (2) erstreckt,
- eine Hülle (10) mit einem ersten Abschnitt (10a), der den Katheter (4) umgibt und aus einem Material besteht, das geeignet ist, Ultraschall und Laserstrahlen durchzulassen, einem zweiten Abschnitt (10b), der den Steuergriff (2) und das Verbindungskabel (3) bis zum Verbindungsstück (1) umgibt, und einem Anschlussstück (11), das zwischen dem ersten und dem zweiten Abschnitt angeordnet ist, wobei das Anschlussstück abnehmbar mit dem Steuergriff (2) verbunden ist.

7. Sonde nach Anspruch 6, wobei der erste Abschnitt (10a) aus einem Polyamid-Polyether-Blockcopolymer besteht.

8. Sonde nach einem der Ansprüche 6 oder 7, wobei der erste Abschnitt (10a) eine konstante Dicke aufweist.

9. Sonde nach einem der Ansprüche 6 bis 8, wobei die Dicke des ersten Abschnitts (10a) zwischen 0,4 und 0,6 mm beträgt.

10. Sonde nach einem der Ansprüche 6 bis 9, wobei der zweite Abschnitt (10b) einen größeren Durchmesser als der erste Abschnitt (10a) aufweist.

11. Sonde nach einem der Ansprüche 6 bis 10, wobei das Anschlussstück eine zentrale Öffnung für den Durchgang des Katheters aufweist, wobei der erste Abschnitt um die zentrale Öffnung des Anschlussstücks herum befestigt ist und der zweite Abschnitt an einem peripheren Abschnitt des Anschlussstücks befestigt ist.

12. Bildgebungssystem mit einer echographischen Station, das eine Laserquelle und eine endoskopische photoakustische Sonde nach einem der Ansprüche 1 bis 11 umfasst, wobei die Sonde mit der Station verbunden ist, wobei die Station einen Prozessor umfasst, der so konfiguriert ist, dass er ein von der Sonde aufgenommenes Bild der Vaskularisierung eines intraartikulären Zielbereichs anzeigt.

13. System nach Anspruch 12, wobei der Prozessor so konfiguriert ist, dass er ein echographisches Bild eines Meniskus und ein photoakustisches Bild der Vaskularisierung des Meniskus überlagert.

## Claims

1. Photoacoustic ultrasound probe (100), comprising:
- a catheter (4),
- an ultrasound transducer (5) fitted to a distal end (40) of the catheter,
- at least one optical fibre (50) adapted to be connected to a laser source, said optical fibre extending into the catheter to the distal end (40),
said probe being **characterised in that** the catheter has an inclined distal portion (42) and/or a bevelled distal end (40).

2. The probe of claim 1, wherein the catheter (4) has a distal portion (42) inclined at an angle of between 10 and 30° with respect to a proximal portion (41) of the catheter.

3. A probe according to any one of claims 1 or 2, wherein the distal end of the catheter (40) is inclined at an angle comprised between 10 and 30° with respect to an axis of revolution of the distal part (42) of the catheter.

4. A probe according to claims 2 and 3 taken in combination, wherein the distal end (40) of the catheter extends in a plane inclined at 30° with respect to the proximal portion (41) of the catheter.

5. A probe according to any of claims 1 to 4, comprising a plurality of optical fibres (50) arranged on either side of the ultrasound sensor (5).

6. A probe according to any of claims 1 to 5, further comprising:
- a control handle (2) attached to the proximal portion (41) of the catheter,
- a connector (1) adapted to be connected to an ultrasound station,
- an electrical connection cable (3) extending between the connector (1) and the handle (2),
- a sheath (10) comprising a first portion (10a) surrounding the catheter (4), made of a material suitable for passing ultrasound and the laser beam, a second portion (10b) surrounding the handle (2) and the cable (3) up to the connector (1), and fitting (11) arranged between the first and second portions, said fitting being removably fixed to the handle (2).

7. A probe according to claim 6, wherein the first portion (10a) is of a polyamide-polyether block copolymer.

8. A probe according to one of claims 6 or 7, wherein the first portion (10a) has a constant thickness.

9. A probe according to any of claims 6 to 8, wherein the thickness of the first portion (10a) is between 0.4 and 0.6 mm.

10. A probe according to any of claims 6 to 9, wherein the second portion (10b) has a larger diameter than the first portion (10a).

11. A probe according to any of claims 6 to 10, wherein the fitting comprises a central opening for the passage of the catheter, the first portion being secured around said central opening of the fitting and the second portion being secured to a peripheral portion of the fitting.

12. An imaging system comprising an ultrasound station including a laser source, and an endoscopic photoacoustic probe according to any of claims 1 to 11 connected to said station, wherein the station comprises a processor configured to display an image of the vascularity of an acquired intra-articular area of interest acquired by the probe.

13. The system of claim 12, wherein the processor is configured to superimpose an ultrasound image of a meniscus and a photoacoustic image of the vascularity of said meniscus.
